# EUROPÄISCHE PATENTSCHRIFT

(11) **EP 1 480 614 B1**
(45) Veröffentlichungstag und Bekanntmachung des Hinweises auf die Patenterteilung: **15.10.2008**
(21) Anmeldenummer: 03717133.7
(22) Anmeldetag: 06.03.2003
(51) Int. Cl.: A61K 9/00, A61K 9/107, A61K 9/51, A61K 38/42

(54) **HYDROPHOBE NANOTROPFEN MIT GEBUNDENEN HÄMOGLOBINMOLEKÜLEN IN HYDROPHILER PHASE ENTHALTENDE EMULSION ALS BLUTERSATZSTOFF**
EMULSION CONTAINING HYDROPHOBIC NANODROPS WITH BOUND HEMOGLOBIN MOLECULES IN A HYDROPHILIC PHASE AS A BLOOD SUBSTITUTE
EMULSION CONTENANT DES GOUTTES HYDROPHOBES DE L'ORDRE DU NANOMETRE AVEC DES MOLECULES D'HEMOGLOBINE LIEES EN PHASE HYDROPHILE, UTILISEE COMME SUBSTITUT DU SANG

(30) Priorität: 06.03.2002 DE 10209860
(43) Veröffentlichungstag der Anmeldung: 01.12.2004
(73) Patentinhaber: Hemofarm Koncern A.D., 26300 Vrsac (YU)
(72) Erfinder: BUGARSKI, Branko, YU-Belgrad (YU); DOVEZENSKI, Nebojsa, YU-Belgrad (YU); STOJANOVIC, Nevenka, YU-Belgrad (YU); BUGARSKI, Diana, YU-Belgrad (YU)
(74) Vertreter: Schüssler, Andrea
(86) Internationale Anmeldenummer: PCT/DE2003/000726
(87) Internationale Veröffentlichungsnummer: WO 2003/074022

(56) Entgegenhaltungen:
- US-A- 5 061 688
- US-A- 5 152 923

## Beschreibung

Die vorliegende Erfindung betrifft eine Öl-in-Wasser-Emulsion, die hydrophobe Nanotropfen in hydrophiler Phase umfaßt, wobei an die Oberfläche der Nanotropfen Hämoglobinmoleküle gebunden oder absorbiert sind und wobei die Hämoglobinmoleküle miteinander vernetzt sind. Diese Emulsion eignet sich vorzugsweise als Blutersatztstoff.

Bei den bisher verwendeten Blutersatzstoffen handelt es sich im wesentlichen um folgende Systeme: (a) Liposomisch gekapseltes Hämoglobin, (b) vernetztes Hämoglobin in wäßriger Phase, (c) multiple Emulsionen wie sie z.B. in dem US-Patent Nr. 5,217,648 beschrieben sind und (d) perfluorierte Kohlenwasserstoffe. US 5,152,923 beschreibt eine Nanoemulsion von Ölpartikeln (aus Triglycerid oder Fettsäureester und amphoterer Emulgator) in einer wässrigen Phase. Die Nanoemulsion ist während mehrerer Monate stabil. Sie kann als Träger für einen Stoff mit sauerstoffübertragender Funktion verwendet werden und damit als Blutersatz dienen. Allerdings weisen die bisher verwendeten Blutersatzstoffe eine Reihe von Nachteilen auf. Dazu zählen beispielsweise nicht ausreichende Biokombatibilität, Toxizität, geringe Kapazität für Sauerstoffbindung einerseits oder zu hohe Affinität für Sauerstoffbindung andererseits, etc.

Somit liegt der Erfindung im wesentlichen das technische Problem zugrunde, Blutersatzstoffe bereitzustellen, die die vorstehenden Nachteile nicht aufweisen.

Die Lösung dieses technischen Problems wurde durch die Bereitstellung der in den Patentansprüchen gekennzeichneten Ausführungsformen erreicht.

Es wurde überraschenderweise gefunden, dass eine "normale" Öl-in-Wasser-Emulsion, die hydrophobe Nanotropfen in hydrophiler Phase enthält, wobei an die Oberfläche der Nanotropfen Hämo-Emulgator) in einer wässrigen Phase. Die Nanoemulsion ist während mehrerer Monate stabil. Sie kann als Träger für einen Stoff mit sauerstoffübertragender Funktion verwendet werden und damit als Blutersatz dienen. globinmoleküle absorptiv gebunden sind und wobei die Hämoglobinmoleküle miteinander vernetzt sind, einen ausgezeichneten Blutersatzstoff darstellt. Darüber hinaus eignet sich die erfindungsgemäße Emulsion allgemein als Sauerstoffträger, als Sauerstoff-"Balancer" in Gewebe- und/oder Zellkultursystemen, in mikrobiologischen Kulturen, bei Organtransplantationen und beim interoperativen Organtransport, bei der Herstellung von Biomasse, als Mittel zur Behandlung von Anämie, zur (legalen) Leistungssteigerung, z.B. bei Piloten, im Militärbereich etc., als therapeutisches Mittel auf Hb-Basis für Effektenmodulation von Stickstoffoxid und zwar zur selektiven Bindung von übermäßigen NO-Mengen in Inflammation oder zur selektiven Lieferung von besonderen No-Formen bei ischemischen Störungen sowie zur Behandlung von Problemen mit Sauerstoffradikalen auf Basis der anti-oxidativen Hb-Eigenschaften.

Aufgrund der Ergebnisse der nachstehenden Beispiele 2 bis 7 zeigte sich, dass die erfindungsgemäße Emulsion folgende vorteilhafte Eigenschaften aufweist:
keine Toxizität und gute Biokompatibiltät, da die Emulsion *in vivo* keine abnormen oder nachteiligen chemischen oder physiologischen Auswirkungen hat; hohe biologische Effizienz; aufrechterhaltene native Struktur und funktionelle Aktivität des gebundenen Hämoglobins im Sinne der Sauerstoffbindung/Freisetzung.

Darüberhinaus haben die biologischen Tests gezeigt, daß die erfindungsgemäße Emulsion auch folgende Wirkungen hat und zwar; sie kann effizient hämodynamische Parameter wiederherstellen, die durch einen hämorrhagischen Schock gestört wurden; sie induziert "Polyzythämie-ähnliches" Syndrom als "Toploaded" bei normalen Tieren, bei massiver 80% Hämatorrhö führt sie zu einer Steigerung der Überlebensrate.

Im Vergleich zu den bisher verwendeten Blutersatzstoffen zeichnet sich die erfindungsgemäße Emulsion u.a. durch folgende Vorteile aus: (a) Sie zeigt einen lang anhaltenden biologischen Effekt, (b) sie wirkt als Erythropoestimulator, (c) für die Herstellung wird kein Hochdruckhomogenisierer benötigt, was insofern vorteilhaft ist, als Hochdruckhomogenisierer mit hohen Scherkräften arbeiten, die Emulsionen leicht zu "echten Lösungen" destabilisieren können, (d) sie ist wesentlich stabiler (auch bei Lagerung), und (e) sie ist einfacher und somit kostengünstiger herzustellen.

Somit betrifft die vorliegende Erfindung eine Emulsion, die hydrophobe Nanotropfen in einer wäßrigen Phase enthält, wobei Hämoglobinmoleküle im wesentlichen an die Oberfläche der hydrophoben Nanotropfen gebunden oder absorbiert sind, wobei die Hämoglobinmoleküle miteinander vernetzt sind.

Der hier verwendete Ausdruck "Emulsion, die hydrophobe Nanotropfen in einer wäßrigen Phase enthält" betrifft ein Nanoemulsionssystem, das im Prinzip aus einem Tropfen Öl besteht, der als hydrophobes Zentrum wirkt, wobei die Oberfläche mit Molekülen bedeckt ist, die amphiphile Eigenschaften aufweisen, z.B. SPAN, Lecithin, Sphyngomyelin, Proteine etc., und zu der umgebenden wäßrigen Phase orientiert sind. Diese amphiphilen Moleküle weisen hydrophobe Schwänze auf, die zu dem hydrophoben Zentrum des Öltropfens orientiert sind und mit diesem interagieren, während die hydrophilen Anteile auf der Oberfläche sind und durch Wasser hydriert werden. Die erfindungsgemäße Emulsion (ohne gebundenes Hämoglobin) kann gemäß dem Fachmann bekannten Verfahren, z.B. mit dem in dem nachfolgenden Beispiel 1 beschriebenen Verfahren hergestellt werden. Der Fachmann kennt auch die verschiedenen Parameter, die bei der Herstellung der erfindungsgemäßen Emulsion beachtet werden müssen.

Für die weitere Herstellung der erfindungsgemäßen Emulsion sollte vorzugsweise hochgereinigtes natives Hämoglobin (frei von angelagertem Sauerstoff) und ohne den alosterischen Inhibitor 2,3-DPG verwendet werden. Die Bindung bzw. Absorption der Hämoglobinmoleküle an die hydrophoben Nanotropfen erfolgt vorzugsweise durch einfaches Vermischen einer Hämoglobinlösung mit der Emulsion. Die Absorption des Hämoglobins an die Oberfläche der Nanotropfen erfolgt durch hydrophobe Interaktionen und nach gegebener Zeit positioniert und orientiert sich der hydrophobe Teil des Moleküls zum hydrophoben Kern des Nanotropfens. Der hydrophile Rest des Hämoglobinmoleküls kann über nicht-kovalente seitliche Bindungen auch mit weiteren Bestandteilen des oberflächlichen, hydrophilen Anteils des Nanotropfens reagieren. Um die korrekte Interaktion zwischen Hämoglobin und Nanotropfen zu gewährleisten, sollte auf die optimale Zusammensetzung der Ölphase geachtet werden, da die chemischen Eigenschaften, Hydrophobizität bzw. amphiphilen Eigenschaften an der Nanoemulsion beteiligten Strukturen eine Auswirkung auf die Bindung der hydrophoben Nische von Hämoglobin haben und somit teilweise als ein "stunt"-2,3-DPG-Moderator wirken. Diese Eigenschaft kann wesentlich dazu beitragen um eine geeignete Affinität des Hämoglobins für Sauerstoff zu erreichen und aufrecht zu erhalten und diese sollte, als p50-Wert ausgedrückt, beim Menschen im Bereich von vorzugsweise etwa 27 mm Hg liegen. Wenn die Komponenten der Nanoemulsion so gewählt sind, dass sie als "stunt"-DPG wirken, ist eine chemische Modifikation der Affinität des Hämoglobins für Sauerstoff nicht erforderlich.

Eine zusätzliche Vernetzung bzw. kovalente Bindung der Hämoglobinmoleküle untereinander führt zu einer erhöhten chemischen und physikalischen Stabilität der erfindungsgemäßen Emulsion. Außerdem wird dadurch im Fall einer intravenösen Injektion die Fähigkeit des Haptoglobins an freies Hämoglobin zu binden verringert.

Die zusätzliche Vernetzung bzw. kovalente Bindung der Hämoglobinmoleküle kann durch dem Fachmann bekannte Verfahren, bei denen die biologische Funktion von Hämoglobin im wesentlichen erhalten bleibt, durchgeführt werden. Dem Fachmann sind Mittel bekannt, die eine kovalente Bindung bzw. chemisches Konjugieren bewirken, beispielsweise kann ein Quervernetzer wie Glutaraldehyd, verwendet werden. Nach der Durchführung der Vernetzungs-bzw. kovalenten Bindungsreaktion sollten eventuell verbliebene freie Moleküle durch geeignete Verfahren entfernt werden. Im letzten Schritt schließlich sollte zur Erzielung der gewünschten Eigenschaften, z.B. für intravenöse Injektion, eine Äquilibrierung des Komplexes mit einem physiologisch verträglichen Medium, vorzugsweise Ringer-Lösung, durchgeführt werden. Diese Schritte können von der technischen Seite gleichzeitig durch eine der Dialysearten, z.B. mit tangentialer Ultadiafiltration, realisiert werden.

Vorzugsweise sollte die erfindungsgemäße Emulsion außerdem eine Verbindung enthalten, die die Oxidation kontrolliert, da die Lipidphase in Gegenwart von freiem Fe⁺⁺⁺ leicht oxidiert. Die Verringerung der Wahrscheinlichkeit der Oxidation von Hämoglobin zu metHämoglobin, in dem Eisen in Form von Fe⁺⁺⁺ vorliegt, ist insofern wichtig, als der weitere Abbau von Hämoglobin zur Freisetzung von Hämin führt und schließlich zum Auftreten von freiem Fe⁺⁺⁺ im Medium, d.h. einer Form von Eisen, die ein hohes Oxidationspotential aufweist. Geeignete Verbindungen zur Kontrolle der Oxidation sind z.B. Vitamin E und C, die entsprechenden Phasen (polaren und nicht-polaren) bei der Gestaltung von Präparaten mit hydrophoben Nanotropfen mit gebundenen Hämoglobinmolekülen in hydrophiler Phase enthaltene Emulsion als Blutersatzstoff zugegeben werden können: Vitamin E während der Emulsionsvorbereitung und Vitamin C in die Zusammensetzung des nativen Hämoglobins. Die Anwesenheit von Vitamin C in wäßriger Phase und nach der endgültigen Gestaltung des Blutersatzstoffes ist aus gleichen Gründen erwünscht.

Die therapeutische Eignung der erfindungsgemäßen Emulsion, z.B. als Blutersatzsoff, und die optimale Konzentration können mit den in den nachstehenden Beispielen 2 bis 7 beschriebenen Verfahren überprüft werden.

In einer bevorzugten Ausführungsform der erfindungsgemäßen Emulsion umfassen die Nanotropfen Pflanzenöl, vorzugsweise Sojabohnenöl, ein Triglyzerid und/oder einen Fettsäurester, ein Phospholipid oder ein Phospholipid-Gemisch, vorzugsweise Phosphotydilcholin, Phosphodilethanolamin und/oder Sphyngomylin.

In einer noch mehr bevorzugten Ausführungsform weisen die hydrophoben Nanotropfen der erfindungsgemäßen Emulsion einen Durchmesser unter 300 nm, am meisten bevorzugt unter 200 nm auf. Für eine intravenöse Injektion sollte der Durchmesser der Nanotropfen jedenfalls unter 200 nm liegen.

Die vorliegende Erfindung betrifft auch ein Arzneimittel, das eine pharmakologisch wirksame Menge einer erfindungsgemäßen Emulsion, gegebenenfalls mit weiteren Hilfsstoffen, enthält. Geeignete Hilfsstoffe und die Formulierung derartiger Arzneimittel sind dem Fachmann bekannt. Die geeignete Dosierung wird von dem behandelnden Arzt bestimmt und hängt von verschiedenen Faktoren ab, beispielsweise von dem Alter, dem Geschlecht, dem Gewicht des Patienten, der Art der Erkrankung oder dem Ausmaß eines Blutverlusts etc.

Die vorliegende Erfindung betrifft schließlich auch die Verwendung einer erfindungsgemäßen Emulsion als Blutersatzstoff im Sinne des Sauerstoffträgers, als Sauerstoff-"Balancer" in Gewebe und/oder Zellkultursystemen, in mikrobiologischen Kultüren, bei Organtransplantationen und beim interoperativen Organtransport, bei der Herstellung von Biomasse, als Mittel zur Behandlung von Anämie, zur (legalen) Leistungssteigerung, z.B. bei Piloten, im Militärbereich etc.; als therapeutisches Mittel auf Hb-Basis für Effektenmodulation von Stickstoffoxid und zwar entweder zur selektiven Bindung von übermäßigen NO-Mengen in Inflammation oder zur selektiven Lieferung von besonderen NO-Formen bei ischemischen Störungen; und zur Behandlung von Problemen mit Sauerstoffradikalen, auf Basis der anti-oxidativen Hb-Eigenschaften.

### Legenden zu den Figuren

### Figur 1: Verteilung von dispergierter Phase der Öl-in-Wasser-Emulsion durch die Vermischungsmethode, wie im Beispiel 1 (H/D=1.9) beschrieben

| | |
|---|---|
| Mittlere Größe | :0.14 µm |
| Durchmesser für 70 % | :0.84 µm |
| Speziflsche Oberäche | :339165 cm2/g |
| 200 < Konzentration < 400 | |

### Figur 2: Verteilung von dispergierter Phase der Öl-in-Wasser-Emulsion durch die Vermischungsmethode, wie im Beispiel 1 (H/D=2.65) beschrieben

| | |
|---|---|
| Durchmesser für 70 % | :0.15 µm |
| Speziflsche Oberäche | :574764 cm2/g |
| 200 < Konzentration < 400 | |

Die nachstehenden Beispiele erläutern die Erfindung. Beispiel 1 bezieht sich auf die Herstellung der erfindungsgemäßen Emulsion, die Beispiele 2 bis 7 beziehen sich auf 3 unterschiedliche Arten von in vivo-Untersuchungen, um die Sicherheit und Effizienz zu überprüfen: (a) Austauschtransfusionsverfahren, bei denen ein großes Blutvolumen gegen ein gleiches Volumen der erfindungsmäßigen Emulsion unter gleichzeitiger Entnahme/Infusion ausgetauscht wurde, so dass die Tiere etwa isovolämisch blieben; (b) Verfahren mit Hämatorrhö und anschließender Reanimation; (c) "Toploading"-Infusion der erfindungsmäßigen Emulsion ohnen Blutentnahme.

### Beispiel 1

### Herstellung der erfindungsgemäßen Emulsion

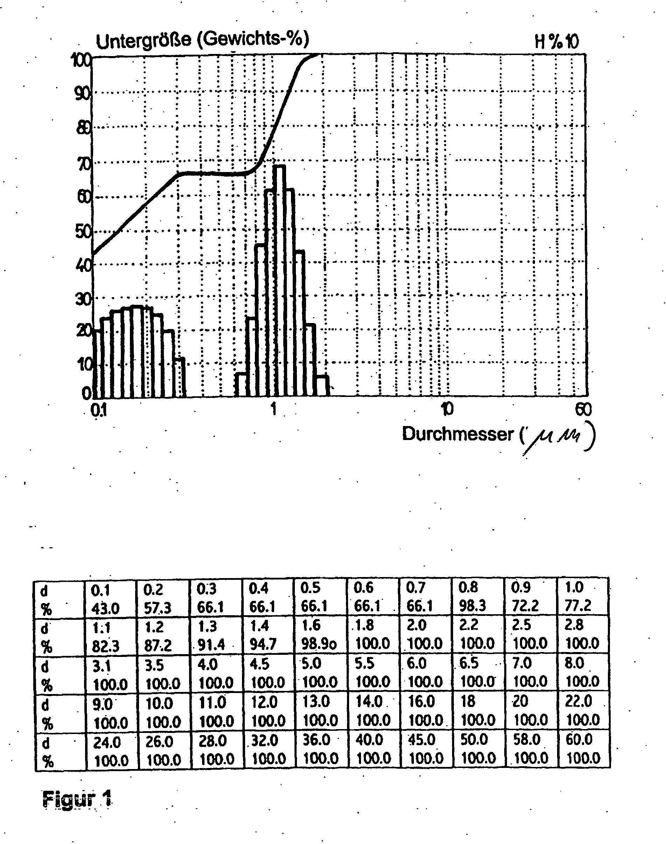

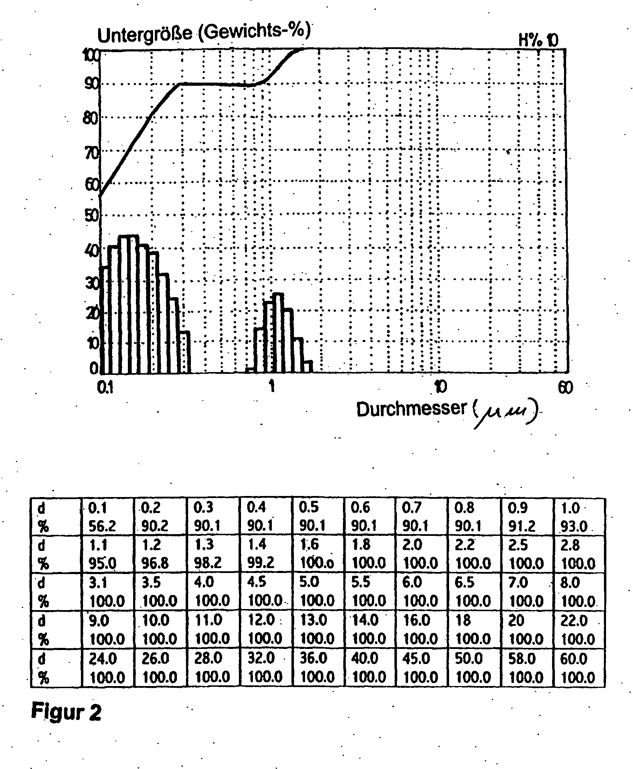

14 g Phospholipide (bestehend aus 85% Phosphotydilcholin aus Sojabohnen oder Eigelb, 8% Phosphotydilethanolamin, 4% Sphyngomylin (alles Emulgatoren), 2% Cholesterin und 1% Triglyzerid wurden mit 875 ml H₂O und 25 ml Glyzerin vermischt. Der benutzte Rührkessel hatte ein Höhen-Durchmesser-Verhältnis von H:D = 1,9 (Fig. 1) bis 2,65 (Fig. 2).

Die Mischung wurde mit einem "Spiralmixer" eine Stunde gerührt, wobei anfänglich mit 100 UpM gerührt wurde_{.} Danach wurde die Rührgeschwindigkeit linear erhöht, wobei am Ende der Rührzeit (nach einer Stunde) eine Rührgeschwindigkeit von 2.000 UpM erreicht wurde.

Danach wurden 100 ml Sojabohnenöl (im gleichen Kessel) dazugegeben. Es folgte eine erneute Rührperiode, diesmal jedoch vier Stunden mit 4.500 UpM. Das erhaltene Produkt ist bzgl. der Größe der Nanotropfen in Fig. 1 und 2 charakterisiert.

Die Mischung (Figur 2) wurde aufgrund des besseren Ertrages von kleinerem Durchmesser der dispergierten Phase gewählt, die dann durch den Porendurchmesser 0,22 *µ*m filtriert wurde. Diese Mischung wurde im Hbbs-Komplex weiter verwendet.

Es wurde die Hämoglobinlösung gemäß dem in DE-PS 197 07 508 beschriebenen Verfahren verwendet. Diese Lösung von nativem und hoch gereinigtem Hämoglobin (Hb) wurde mit der gefilterten Emulsion in einem Volumenverhältnis von 1:1 vermischt. Dann wurde eine Glutaraldehydlösung = 1: 11 zugegeben. Es folgte eine weitere Rührperiode, diesmal 30 bis 50 Minuten mit 10 bis 35 UpM und im Anschluß danach wurde die Mischung gegen Ringer-Lösung dialysiert, um sie isotonisch zu machen und den Überschuß von Glutaraldehyd zu entfernen.

### Beispiel 2: In vivo-Evaluierung der erfindungsgemäßen Emulsion (Hbbs) - 30% isovolämische Austauschtransfusion zur Beurteilung der allgemeinen Toxizität sowie der Fähigkeit der erfindungsgemäßen Emulsion, im Falle schwerer Anämie entsprechende hämodynamische Parameter aufrechtzuerhalten.

Es wurden Wistar-Ratten (230 - 250 g Körpergewicht) verwendet. Am Morgen des Experiments wurden die Ratten zur Bestimmung des Gesamtblutvolumens gewogen und anästhesiert. In die Halsschlagader (zur Blutentnahme) und die Oberschenkelvene (zur Blutinfusion) wurden Kanülen gelegt. Die Austauschtransfusion wurde manuell durchgeführt, wobei das Blut mit einer Rate von 1 ml/2 Min. entfernt/transfusioniert wurde.

Vier Gruppen von Ratten wurden einer Austauschtransfusion (30% des Blutvolumens) unterzogen:
I. Bei einer Gruppe wurde ein Drittel des geschätzten Blutvolumens durch Hbbs ersetzt (600-900 mg Hb, gebunden an die Emulsion/ kg Körpergewicht).

### Kontrollgruppen:

II. Bei einer Gruppe wurde ein Drittel des geschätzten Blutvolumens durch Saline ersetzt.
III. Bei einer Gruppe wurde ein Drittel des geschätzten Blutvolumens durch Emulsion ohne Hb ersetzt.
IV. Bei einer Gruppe wurde ein Drittel des geschätzten Blutvolumens durch reines Hb ersetzt, das nicht an die Emulsion gebunden war (600-900 mg Hb/kg Körpergewicht).

Die Veränderungen der hämatologischen Parameter (peripheres Blut), hämodynamischen Parameter und Blutgasparameter wurden analysiert. Die hämatologischen Parameter (peripheres Blut)(Gesamtzahl und Einzelwerte der weißen Blutzellen, Blutplättchen, Erythroyzyten, Retikulozyten, Hämatokrit (Hct) und Hämoglobinkonzentration) wurden 0 Min. (Basislinie), 10, 20, 30, 60 und 120 Min. nach Austauschtransfusion bestimmt. Die hämodynamischen Parameter wurden in anästhisierten Ratten bestimmt, bei denen der mittlere arterielle Blutpdruck (MAP), der Puls (HR) und der Herz-Output (CO) sowie die gesamte periphere vaskuläre Resistenz (TVR) berechnet wurden, und zwar nach 0 Min. (Basislinie), nach Blutentnahme, nach Flüssigkeitsinfusion, 10, 20, 30, 60 und 120 Min. nach Austauschtransfusion. Die Blutgasparameter (arterieller und venöser pO₂, Prozentsatz des Sauerstoffgehalts, pCO₂, CO₂-Gehalt, pH-wert, p50) wurden nach 0 Min. (Basislinie), nach Blutentnahme, 10, 20, 30, 60 und 120 Min. nach Austauschtransfusion bestimmt.

### Ergebnisse

### Veränderungen der hämatologischen Parameter (peripheres Blut)

Die Entfernung von 30% Blut führte im wesentlichen zu ähnlichen Änderungen in allen getesteten Rattengruppen. Die Hb-Konzentration und die Hct-Werte nahmen in allen Gruppen um eine vergleichbare Größenordnung ab, wie dies auch nach Blutentnahme erwartet wurde. Trotz individueller Unterschiede änderten sich die Gesamtzahl und die Einzelwerte von Leukozyten, roten Blutkörperchen, Retikulozyten und Blutplättchen nicht signifikant innerhalb der getesteten Gruppen und diese änderten sich auch nicht nach Hbbs-Austauschtransfusion.

### Veränderungen der hämodynamischen Parameter

30% Hämatorrhö war durch einen MAP-Abfall bis zu 80%, einen CO-Abfall bis zu 65% und einen VTR-Anstieg bis zu 50% charakterisiert. Die Austauschtransfusion mit Hbbs war hinsichtlich der Wiederherstellung der hämodynamischen Parameter am wirkungsvollsten. MAP war innerhalb der ersten 10 Min. etwas erhöht (etwa 20% über normal), hielt bis zu 20 Min. an und kehrte bei 30 Min. zur Basislinie zurück. CO war zeitgleich erhöht, kehrte jedoch nach 30 Min. zur Basislinie zurück. TVR und HR änderten sich nicht während des Beobachtungszeitraums. In Saline und reiner Emulsion fielen bei den behandelten Gruppen die Werte für MAP und CO ab und kehrten nach 120 Min. zur Basislinie zurück, TVR und HR änderten sich nicht. Die Verabreichung von reinem Hb induzierte einen erhöhten MAP, einen um bis zu 50% verringerten CO und einen erhöhten TVR, was auf gefäßverengende Wirkungen hindeutet.

### Veränderungen der Blutgasparameter

Nach 30% Hämatorrhö stieg der arterielle PO₂, dieser kehrte jedoch nach Behandlung mit Hbbs innerhalb von 20 Min. zur Basislinie zurück - im Gegensatz zu konstant niedrigeren Spiegeln nach Austauschtransfusion mit einer reinen Hämoglobinlösung, d.h. nicht an Emulsion gebundenem Hb. Nach 30% Hämatorrhö stieg der venöse PO₂, dieser kehrte jedoch nach Behandlung mit Hbbs innerhalb von 20 Min. zur Basislinie zurück - im Gegensatz zu konstant niedrigeren Spiegeln nach Austauschtransfusion mit einer reinen Hämoglobinlösung, d.h. nicht an Emulsion gebundenem Hb. Nach Berechnung der O₂-Sättigung und des Sauerstoffgehalts zeigten sich innerhalb der Gruppen keine Unterschiede, lediglich in venösem Blut wurde nach Austauschtransfusion mit reiner Hämoglobin-Lösung ein höherer Sauerstoffgehalt ermittelt. Die p50-Werte verringerten sich nach Austauschtransfusion aufgrund der Hämodilution, kehrten jedoch innerhalb von 2 Stunden zur Basislinie zurück.

### Beispiel 3: In vivo-Evaluierung der erfindungsgemäßen Emul sion(Hbbs) - 50% isovolämische Austauschtransfusion zur Beurteilung der allgemeinen Toxizität sowie der Fähigkeit der erfindungsgemäßen Emulsion, im Falle schwerer Anämie entsprechende hämodynamische Parameter aufrechtzuerhalten.

Es wurden Wistar-Ratten (180 - 220 g Körpergewicht) verwendet. Am Morgen des Experiments wurden die Ratten zur Bestimmung des Gesamtblutvolumens gewogen und anästhesiert. In die Halsschlagader (zur Blutentnahme) und die Oberschenkelvene (zur Blutinfusion) wurden Kanülen gelegt. Die Tiere wurden einer 50% Blutvolumen-Hämatorrhö unterzogen, danach erfolgte eine Hbbs-Transfusion mit einer Rate von 1 ml / 30 Min. (Infusionspumpe).

Zwei Gruppen von Ratten wurden einer (50% Blutvolumen-Austauschtransfusion) unterzogen:
I. Bei einer Gruppe wurden 50% des geschätzten Blutvolumens durch Hbbs ersetzt (450-550 mg Hb, gebunden an die Emulsion/kg Körpergewicht, was etwa 15% des durch die Blutentnahme verlorenen Hämoglobins entspricht).
II. Kontrollgruppe:

Bei einer Gruppe wurden 50% des geschätzten Blutvolumens durch Ringer-Lösung ersetzt.

Die Veränderungen der hämatologischen Parameter und hämodynamischen Parameter wurden analysiert. Die hämatologischen Parameter (peripheres Blut)(Gesamtzahl und Einzelwerte der weißen Blutzellen, Blutplättchen, Erythroyzyten, Retikulozyten, Hämatokrit und Hämoglobinkonzentration) wurden 0 Min. (Basislinie) und 1 Stunde nach Austauschtransfusion bestimmt. Die hämodynamischen Parameter (systolischer Blutdruck, dyastolischer Blutdruck, mittlerer arterieller Blutdruck (MAP) und Puls (HR)) wurden nach 0 Min. (Basislinie), nach Blutentnahme, nach Hbbs-Austauschtransfusion oder Ringer-Lösung-Infusion und 1 Stunde nach Austauschtransfusion bestimmt.

### Ergebnisse

1 Stunde nach Austauschtransfusion verringerten sich die Hct-Werte, Hb-Konzentrationen, die Anzahl der roten Blutkörperchen und der Blutplättchen in beiden Gruppen, was einem Blutverlust von 50% Prozent und der Hämodilutions-Wirkung entspricht. Der arterielle Blutdruck und der Puls verringerten sich nach 50% Blutverlust und Blutaustausch gegen Hbbs signifikant, beide Parameter kehrten jedoch nach 1 Stunde zur Basislinie zurück. Nach Austausch gegen Ringer-Lösung lagen jedoch sowohl MAP als auch HR 1 Stunde nach der Austauschtransfusion unter den Werten der Basislinie.

### Beispiel 4: In vivo-Evaluierung der erfindungsgemäßen Emul sion (Hbbs) - 80% isovolämische Austauschtransfusion zur Beurteilung der biologischen Effizienz sowie der erfindungsgemäßen Emulsion im Falle einer schweren Anämie.

Es wurden Wistar-Ratten (300 - 360 g Körpergewicht) verwendet. Am Morgen des Experiments wurden die Ratten zur Bestimmung des Gesamtblutvolumens gewogen und anästhesiert. In die Halsschlagader (zur Blutentnahme) und die Oberschenkelvene (zur Blutinfusion) wurden Kanülen gelegt. Danach wurden die Tiere weiter unter Anästhesie gehalten und einer 80% Blutvolumen-Hämatorrhö unterzogen, danach erfolgte unverzüglich ein Austausch von etwa 75% des entfernten Blutes gegen alogenes Plasma bei einer Rate von 0,5 ml /Min. danach gegen Hbbs (bei einer Rate von 1 ml / 30 Min.)

Vier-Gruppen von Ratten wurden einer Austauschtransfusion (80% Blutvolumen-Hämotorrhoe) unterzogen:
I. Bei einer Gruppe wurden 80% des geschätzten Blutvolumens durch Hbbs ersetzt (880 - 1050 mg Hb, gebunden an die Emulsion/ kg Körpergewicht). Kontrollgruppen:
II. Bei einer Gruppe wurden 80% des geschätzten Blutvolumens durch alogenes Plasma + Ringer-Lösung ersetzt.
III. Bei einer Gruppe wurden 80% des geschätzten Blutvolumens durch alogenes Plasma + autologes Blut ersetzt.
IV.Bei einer Gruppe wurden 80% des geschätzten Blutvolumens durch alogenes Plasma ersetzt.

Die Überlebensraten und Veränderungen der Hämotokrit-Werte, Hämoglobin-Konzentration und der p50-Werte wurden vor und unmittelbar nach Blutentnahme und Austausch untersucht.

### Ergebnisse

Nach 80% Hämatorrhö wurden verringerte Hct-Werte (bis zu 75%) und Hämoglobin-Konzentrationen (bis zu 70%)gemessen. Die Tiere aller vier Gruppen zeigten eine Überlebensrate von 100% während der Austauschtransfusion, die Überlebensrate fiel jedoch während 24 Stunden aufgrund der Dauer der Anästhesie steil ab. Es zeigten sich keine Unterschiede in der Überlebensrate bei den Ratten, bei denen ein Austausch gegen HHbs und autologes Blut erfolgte, da diese beiden Gruppen von Tieren die höchsten (und gleichen) Überlebensraten zeigten. Die geringste Überlebensrate zeigte sich bei Tieren, bei denen eine Austauschtransfusion mit Ringer-Lösung durchgeführt worden war.

### Beispiel 5: In vivo-Evaluierung der erfindungsgemäßen Emulsion (Hbbs) - 60% Hämatorrhö, danach Reanimation zur Beurteilung der Wirksamkeit der erfindungsmäßigen Emulsion bei der Behandlung von akutem Blutverlust und Schock

Es wurden Wistar-Ratten (180 - 220 g Körpergewicht) verwendet. Mindestens zwei Tage vor den Hämatorrhö/Reanimations-Experimenten wurde in jede Ratte ein Dauerkatheter in die Jugular-Vene implantiert. Das Verbindungsröhrchen für den Katheter wurde unter die Rückenhaut und links am Hals gelegt. An dieser Stelle wurde die Katheter-Verbindung befestigt. Am Morgen des Experiments wurden die Ratten zur Bestimmung des Gesamtblutvolumens gewogen, anästhesiert und in die Oberschenkelvene wurden zur Blutentnahme Kanülen gelegt.

Die Ratten wurden einer 60% Blutvolumen-Hämatorrhö unterzogen, danach erfolgte sofort der Austausch von etwa 50% des entfernten Blutes gegen alogenes Plasma bei einer Rate von 0,5 ml /-Min als erster Schritt der Austauschtransfusion. Zur Bewertung von Hbbs als Blutersatz wurden Hbbs und Ringer-Lösung bei einer Rate von 0,125 ml / Min. (Infusionspumpe) über die Dauerkanüle den Ratten bei vollem Bewußtsein infundiert.
I. Bei einer Gruppe mit 60% geschätzter Blutvolumen-Hämatorrhö erfolgte Austausch durch alogenes Plasma + Hbbs(450-1000 mg Hb, gebunden an die Emulsion/ kg Körpergewicht, was etwa 15% des Hämoglobins entspricht, das bei der Blutentnahme verloren ging.)
II. Bei einer Gruppe mit 60% geschätzter Blutvolumen-Hämatorrhö erfolgte Austausch gegen alogenes Plasma + Ringer-Lösung.
III. Bei einer Gruppe mit 60% geschätzter Blutvolumen-Hämatorrhö erfolgte kein Austausch.
IV. Bei einer Gruppe erfolgte Legen einer Dauerkanüle, jedoch keine Hämatorrhö.
V. Normale, nicht behandelte Ratten.

### An den Ratten wurden die folgenden Parameter analysiert: Mortalität und Morbidität

Es wurde auf die Überlebensrate der Tiere, auf die Entwicklung eines anaphylaktischen Schocks, auf die Entwicklung neurologischer Defizite, auf Bronchialkrämpfe oder Lungenödeme, Fieber, Hämoglobinurie, Fehlen normaler Aktivität, Unterdrückung normaler Funktionen/Fressen oder Trinken geachtet.

### Veränderungen hämatologischer Parameter (peripheres Blut)

Die Gesamtzahl und Einzelwerte der weißen Blutzellen, Blutplättchen, Erythroyzyten, Retikulozyten, Hämatokrit und Hämoglobinkonzentration wurden 0 Min. (Basislinie), 24 Stunden, 7, 11 und 30 Tage nach Hämatorrhö/Reanimation bestimmt.

### Veränderungen der hämodynamischen Parameter

Der systolische Blutdruck und der Puls wurden nach 0 Min. (Basislinie) und 24 Stunden nach Hämatorrhö/Reanimation bestimmt.

### Änderungen der Blutgasparameter

PO₂, Sauerstoffsättigung, pCO₂, CO₂-Gehalt, pH-Wert und p50 wurden nach 0 Min. (Basislinie), nach Hämatorrhö, nach Reanimation, 24 Stunden, 7, 11 und 30 Tage nach Hämatorrhö/Reanimation bestimmt.

### Änderungen hämatologischer Parameter in Knochenmark und Milz

Die Gesamtzahl kernhaltiger Zellen und die unterschiedliche Anzahl morphologisch erkennbarer Zellen, die Anzahl von CFU-GM-, BFU-E- und CFU-E-Progenitorzellen wurde 7, 11 und 30 Tage nach Hämatorrhö/Reanimation bestimmt.

### Makroskopische Pathologie und Histopathologie

Bei der Autopsie wurde vor allem auf Hb-Extravasation, makroskopische Anzeichen von Ödemen, Blutandrang, Hämatorrhö und Organinfarkte von Herz, Lungen, Leber, Milz und Nieren geachtet und histopathologische Schnitte von Herz, Lunge, Leber, Milz und Nieren wurden für Lichtmikroskopie aufbereitet. Proben, die 7, 11 und 30 Tage nach Hämatorrhö/Reanimation erhalten worden waren, wurden mittels Lichtmikroskop analysiert.

### Ergebnisse

Ratten mit Hbbs-Infusionen zeigten eine Überlebensrate von 100% sowohl während der Transfusion als auch 24 nach Hämatorrhö/Reanimation. Im Vergleich dazu zeigten Ratten mit Ringer-Lösung-Infusionen eine Überlebensrate von 71,4% während der Transfusion und nur 42,8% bei 24 Stunden. Die Langzeit-Überlebensrate (bis zu 30 Tagen) der mit Hbbs reanimierten Ratten betrug 50%, im Vergleich dazu betrug die Überlebensrate 40% bei Ringer-Lösung. Keines der Tiere überlebte 60% Hämatorrhö ohne Reanimation. Die überlebenden Ratten mit Hbbs-Infusionen waren bei 24 und 48 Stunden beweglich und reagierten auf Stimuli normal.

In Ratten mit Infusion der erfindungsgemäßen Emulsion waren nach einem anfänglichen Abfall aufgrund der 60% Hämatorrhö und der Hämodilution der Hct-Wert, die Hämoglobinkonzentration, die Anzahl der weißen Blutkörperchen und der "differential count", die Anzahl der roten Blutkörperchen und der Blutplättchen am 7. Tag nach Hämodilution im normalen Bereich. Am 7. Tag nach Hämatorrhö/Reanimation konnte jedoch in Ratten mit der erfindungsgemäßen Emulsion ein signifikanter Anstieg der Zahl an unreifen Milz-Erythroid-Progenitorzellen (BFU-E) und reifen Erythroid-Progenitorzellen (CFU-E) sowie an morphologisch erkennbaren Erythroidzellen (Erythroblasten und orthochromatische Erythroblasten) beobachtet werden was ein Anzeichen von Erythropoese ist. Der signifikante Anstieg des Prozentsatzes an peripheren Blutretikulozyten bis zum 11. Tag nach Hämatorrhö/Reanimation in dieser Gruppe von Ratten belegt, dass eine effiziente Erythropoese eintrat. Stimulierte Erythropoese impliziert, dass eine erfindungsgemäße Emulsion vorhanden ist sowie daß durch die erfindungsgemäße Emulsion verfügbar gemachtes Eisen (aus Hb)auf die Stimulation dieses Prozesses wirkt.

Die Untersuchungen über makroskopische Pathologie und Histopathologie ergaben bis zu 30 Tagen nach Hämatorrhö/Reanimation keine signifikanten, durch die erfindungsgemäße Emulsion eventuelle bedingten Veränderungen, was ein Hinweis auf das Fehlen einer Langzeittoxizität der erfindungsgemäßen Emulsion ist.

### Beispiel 6: In vivo-Evaluierung der erfindungsgemäßen Emulsion (Hbbs) - Toploading"-Infusion von Hbbs ohne Blutentnahme zur Beurteilung von spezifischen Toxinitärfragen, d.h. möglichen toxischen Nebenwirkungen

Zur Evaluierung der Sicherheit der erfindungsgemäßen Emulsion würden Ratten mit normalem Blutvolumen bei vollem Bewußtsein 1000 mg an die Emulsion gebundenes Hämoglobin pro kg Körpergewicht als *i*.*v*.-Bolus injiziert (etwa 40% des geschätzten Blutvolumens). Als Kontrollgruppe wurden Ratten verwendet, denen Saline, Emulsion ohne Hb und nicht an die Emulsion gebundenes Hämoglobin (1000 mg/kg Körpergewicht)injziert wurde.

### An diesen Ratten wurden die folgenden Parameter analysiert: Mortalität und Morbidität

Es wurde auf die Überlebensrate, auf die Entwicklung eines anaphylaktischen Schocks, auf die Entwicklung neurologischer Defizite, auf Bronchialkrämpfe oder Lungenödeme, Fieber, Hämoglobinurie, Fehlen normaler Aktivität, Unterdrückung normaler Funktionen/Fressen oder Trinken geachtet.

### Veränderungen hämatologischer Parameter (peripheres Blut)

Die Gesamtzahl und Einzelwerte der weißen Blutzellen, Blutplättchen, Erythroyzyten, Retikulozyten, Hämatokrit und Hämoglobinkonzentration wurden 0 Stunden (Basislinie), 30 Min., 3 Stunden, 24 Stunden sowie 30 und 60 Tage nach Injektion bestimmt.

### Änderungen der Blutgasparameter

pO₂, Sauerstoffsättigung, PCO₂, CO₂-Gehalt, pH-wert und p50 wurden nach 0 Min. (Basislinie), 30 Min., 3 Stunden, 24 Stunden sowie 30 und 60 Tage nach Injektion bestimmt.

### Änderungen hämatologischer Parameter in Knochenmark und Milz

Die Gesamtzahl kernhaltiger Zellen und die unterschiedliche Anzahl morphologisch erkennbarer Zellen, die Anzahl von CFU-GM-, BFU-E- und CFU-E-Progenitorzellen wurde 30 und 60 Tage nach Injektion bestimmt.

### Makroskopische Pathologie und Histopathologie

Bei der Autopsie wurde vor allem auf Hb-Extravasation, makroskopische Anzeichen von Ödemen, Blutandrang, Hämatorrhö und Organinfarkte von Herz, Lungen, Leber, Milz und Nieren geachtet und histopathologische Schnitte von Herz, Lunge, Leber, Milz und Nieren wurden für Lichtmikroskopie aufbereitet. Proben, die 30 und 60 Tage nach Injektion erhalten worden waren, wurden mittels Lichtmikroskop analysiert.

### Ergebnisse

Die erfindungsgemäße Emulsion führte nicht zu Mortalität und während des Beobachtungszeitraums zu keinen klinisch relevanten Befunden.

Der Hct-Wert, die Hämoglobinkonzentration, die Zahl der weißen Blutkörperchen und die differentielle Zählung, die Zahl der roten Blutkörperchen und der Blutplättchen änderte sich nach Injektion der erfindungsgemäßen Emulsion nicht signifikant und es gab auch bei den untersuchten Intervallen keine signifikanten Unterschiede zwischen den einzelnen Gruppen.

24 Stunden nach Injektion der erfindungsgemäßen Emulsion konnten keine Veränderungen der Blutgasparameter beobachtet werden. 30 Tage nach deren Injektion wurden jedoch im Vergleich zu den Kontrollgruppen angestiegene pO₂- und p50-Werte bei Hbbs-behandelten Tieren beobachtet. Diese höhere O₂-Bindung begleitet von einer geringeren Hämoglobinsauerstoff-Affinität war offensichtlich durch die Anwesenheit der erfindungsgemäßen

Emulsion und deren verlängerte Wirkung bedingt.

Zur gleichen Zeit, d.h. 30 Tage nach Injektion der erfindungsgemäßen Emulsion, wurden innerhalb der Erythroidzellen im Knochenmark und der Milz der Ratten, denen die erfindungsgemäße Emulsion injiziert worden war, wichtige Veränderungen beobachtet. Im Knochenmark deutete die verringerte Zahl an reifen Erythroid-Progenitorzellen (CFU-E) und morphologisch erkennbaren Erythroidzellen/orthochromatischen Erythroblasten auf eine unterdrückte Erythropoese hin. Dieses Phänomen wird normalerweise in Polyzythämie-ähnlichen Fällen beobachtet und könnte durch das verlängerte endogene Hyperoxiestadium erklärt werden, was in den Hbbs-behandelten Ratten durch die verlängerte Wirkung der erfindungsgemäßen Emulsion hervorgerufen wurde. Polyzythämie-ähnliches Symptom weist auf "Feedback"-Reaktion hin, die durch verlängerte Hbbs-Wirkung hervorgerufen wurde, wobei Hbbs als "Überschuß an Erythrozyten" die Unterdrückung der Erythropoese im Knochenmark verursacht.

Am 30. Tag wurde in der Milz von Ratten, denen die erfindungsgemäße Emulsion injiziert worden war, eine signifikante Stimulation der Erythropoese beobachtet, was sich durch die erhöhte Zahl sowohl an Erythroid-Progenitorzellen als auch morphologisch erkannbaren Erythroidzellen manifestierte, was erneut impliziert, dass das durch die erfindungsgemäße Emulsion verfügbar gemachte Eisen die Erythropoese unterstützt.

Die Untersuchungen über makroskopische Pathologie und Histopathologie zeigten 30 Tage nach Injektion keine signifikanten, durch die erfindungsgemäße Emulsion eventuelle bedingte Veränderungen.

60 Tage nach Injektion der erfindungsgemäßen Emulsion konnten keine signifikanten Änderungen hinsichtlich aller getesteten Parameter (einschließlich makroskopischer Pathologie und histopathologischer Untersuchungen) nachgewiesen werden, was das Fehlen einer Langzeit-Toxizität der erfindungsgemäßen Emulsion belegt.

### Beispiel 7

### Untersuchungen zur O₂-Bindung/Freisetzung

Die Fähigkeit der erfindungsgemäßen Emulsion zur O₂-Bindung/Freisetzung in vitro wurde bei definierten Bedingungen bekannter Konzentrationen an Sauerstoff, Kohlendioxid und Stickstoff durch Messungen der PO₂-, PCO₂, pH- und p50-Werte evaluiert. Gemäß diesen Ergebnissen in vitro für O₂-Bindung/Freisetzung sowie den in vivo-Messungen der Blutgasparameter die in vorstehenden Beispielen gezeigt wurden, wurde auch gezeigt, daß die native Struktur und funktionelle Aktivität von Hämoglobin in der erfindungsgemäßen Emulsion erhalten blieben und daß sie dem Hämoglobin in humanem Blut entsprechen.

## Patentansprüche

1. Emulsion, enthaltend hydrophobe Nanotropfen in einer wässrigen Phase, wobei Hämoglobinmoleküle im Wesentlichen an der Oberfläche der hydrophoben Nanotropfen gebunden oder absorbiert sind, wobei die Hämoglobinmoleküle miteinander vernetzt sind.

2. Emulsion nach Anspruch 1, wobei die wässrige Phase Ringer-Lösung ist.

3. Emulsion nach Anspruch 1 oder 2, wobei die Nanotropfen Pflanzenöl, ein Triglyzerid und/oder einen Fettsäureester umfassen.

4. Emulsion nach einem der Ansprüche 1 bis 3, wobei der Fettsäureester ein Phospholipid oder ein Phospholipid-Gemisch ist.

5. Emulsion nach einem der Ansprüche 1 bis 4, wobei die hydrophoben Nanotropfen einen Durchmesser unter 300 nm aufweisen.

6. Emulsion nach einem der Ansprüche 1 bis 5, wobei die hydrophoben Nanotropfen einen Durchmesser unter 200 nm aufweisen.

7. Arzneimittel, umfassend eine pharmakologisch wirksame Menge einer Emulsion nach einem der Ansprüche 1 bis 6.

8. Verwendung einer Emulsion nach einem der Ansprüche 1 bis 6 zur Herstellung eines Arzneimittels für therapeutische Anwendungen, nämlich als Blutersatzstoff, Sauerstoffträger, Sauerstoff-"Balancer", bei der Herstellung von Biomasse, zur Behandlung einer Anämie, zur Leistungssteigerung, zur Therapie auf Hb-Basis für Effektenmodulation von Stickstoffoxid oder zur Behandlung von Problemen mit Sauerstoffradikalen.

9. In vitro-Verwendung einer Emulsion nach einem der Ansprüche 1 bis 6 in Gewebe-und/oder Zellkultursystemen oder in mikrobiologischen Kulturen.

## Claims

1. An emulsion comprising hydrophobic nano-drops in an aqueous phase, wherein hemoglobin molecules are bonded or absorbed substantially on the surface of the hydrophobic nano-drops, wherein the hemoglobin molecules are bonded with each other.

2. The emulsion according to claim 1, wherein the aqueous phase is a Ringer solution.

3. The emulsion according to claims 1 or 2, wherein the nano-drops comprise vegetable oil, a triglyceride and/or a fatty acid ester.

4. The emulsion according to any of the claims 1 to 3, wherein the fatty acid ester is a phospholipid or a mixture of phospholipids.

5. The emulsion according to any of claims 1 to 4, wherein the hydrophobic nano-drops have a diameter smaller than 300 nm.

6. The emulsion according to any of the claims 1 to 5, wherein the hydrophobic nano-drops have a diameter smaller than 200 nm.

7. A medicament, comprising a pharmaceutically efficient amount of the emulsion according to any of the claims 1 to 6.

8. An use of the emulsion according to any of the claims 1 to 6, for the preparation of a medicament for therapeutic application, namely as a blood substititute, a carrier of oxygen, an oxygen balancer, for producion of biomass, for treatment of anemia, for increase in performance, for therapy based on Hb for modulating the effects of nitrogen monoxide or for treatment of problems with oxygen radicals.

9. An in vitro application of the emulsion according to any of the claims 1 to 6, for tissue cultures and/or cell cultures, or for microbiological cultures.

## Revendications

1. Emulsion, contenant les nano-gouttes hydrophobes en phase aqueuse, alors que les molécules d'hémoglobine sont en général liées ou absorbées sur la surface des nano-gouttes hydrophobes, pendant que les molécules d'hémoglobines sont mutuellement liées.

2. L'emulsion selon la revendication 1, dans laquelle la phase aqueuse est la solution Ringer.

3. L'emulsion selon la revendication 1 ou 2, dans laquelle les nano-gouttes comprenant de l'huile végétale, du triglycéride et/ou de l'ester de l'acide gras.

4. L'emulsion selon l'une des revendications 1 à 3, dans laquelle l'ester de l'acide gras est un phospholipide ou un mélange de phospholipides.

5. L'emulsion selon l'une des revendications 1 à 3, dans laquelle les nano-gouttes hydrophobes possèdent un diamètre inférieur à 300nm.

6. L'emulsion selon l'une des revendications 1 à 5, dans laquelle les nano-gouttes hydrophobes possèdent un diamètre inférieur à 200nm.

7. Médicament, comprenant une quantité pharmacologique efficace d'émulsion selon l'une des revendications 1 à 6.

8. Utilisation de l'émulsion selon l'une des revendications 1 à 6, pour la fabrication du médicament pour l'application thérapeutique, à savoir comme substitut de sang, porteur d'oxygène, l'oxygène-»Balancer«, comme producteur de la bio-masse, pour le traitement de l'anémie, l'augmentation des capacités, pour la thérapie sur la base du Hb pour la modulation de l'effet du monoxyde de l'azote, et pour le traitement des problèmes avec les radicaux d'oxygène.

9. L'utilisation in vitro de l'émulsion selon l'une des revendications 1 à 6 dans les systèmes de tissus et/ou des cultures de cellule ou dans les cultures microbiologiques.
